# EUROPEAN PATENT SPECIFICATION

(11) **EP 3 171 823 B1**
(45) Date of publication and mention of the grant of the patent: **13.06.2018**
(21) Application number: 15763992.3
(22) Date of filing: 22.07.2015
(51) Int. Cl.: A61F 2/30

(54) **PROTHESIS FOR TEMPOROMANDIBULAR JOINT**
PROTHESE FÜR KIEFERGELENK
PROTHÈSE POUR UNE ARTICULATION TEMPOROMANDIBULAIRE

(30) Priority: 23.07.2014 TR 201408718
(43) Date of publication of application: 31.05.2017
(73) Proprietor: Tosun, Zekeriya, 42000 Konya (TR)
(72) Inventor: TOSUN, Zekeriya, Konya (TR); SEVER, Cem, Konya (TR); AKDAG, Osman, Konya (TR); SELIMOGLU, Muhammed Nebil, Konya (TR); IMIROGLU, Halil Ibrahim, Konya (TR); KAYMAZ, Irfan, Erzurum (TR)
(74) Representative: 2s-ip Schramm Schneider Bertagnoll Patent- und Rechtsanwälte Part mbB
(86) International application number: PCT/TR2015/050045
(87) International publication number: WO 2016/014006

(56) References cited:
- EP-A1- 0 628 293
- WO-A1-2014/043452
- US-A- 6 132 466
- US-A1- 2009 222 102

## Description

### TECHNICAL FIELD

The present invention relates to joint prosthesis and is particularly directed to temporomandibular joint which could not fulfill the task for replacement due to disorder/uncorrectable disorder.

### PRIOR ART

Temporomandibular joint is a dynamic structure which consists of articular disc, ligament ant muscles and is capable of two ways movement with condyle of mandible and a fossa in the temporal bone (skull). Modern society has greatly increased stress effect on our body. One of the most common sites of its devastating effects is on the jaw joint. Painful clicking, dislocation of disc and locking could occur during the daily functions of TMJ such as masticating, yawning or talking and it may reduce our quality of life. Surgical treatment of TMJ disorder is also one acceptable (or preferred) treatment method. The movements of conventional joint prostheses used in TMJ surgery are not the same movements of normal TMJ. Even these prostheses do not have capability of minimal transverse movements called translational movements. Therefore movements of conventional joint prostheses are not similar the physiologic movement of joint so patient comfort cannot be provided after surgery.

Friction is between polyethylene surface of fossa-eminence part and metal surface of mandible part in most of conventional prostheses. Forces during the movements are eliminated by polyethylene surface than the friction is decrease with metal surface. The force transferred to polyethylene part is point force because of geometrical shape of the head of mandibular part. Thus the metal part of prostheses can be deformed more easily. Polyethylene surface is eroded due to friction over and it can become even dysfunctional.

Polyethylene materials are exposed to high pressure due to point forces, so the deformation depending on not only to frictional force. High pressure increases the frictional forces so it will accelerate the deformation speed.

Existing prostheses include metal structures that mimic the fossa and a circular structure that mimic the structure of the mandibular condyle. The adaptation of the existing jaw prosthesis apparatus is very difficult due to person-specific variability of fossa structures. This situation causes the physician to remain longer in operation and also causes some difficulties in the adaptation of the jaw prosthesis.

EP 0 628 293 A1 relates to an implant according to the preamble of claim 1.

### BRIEF DESCRIPTION OF THE INVENTION

The object of the present invention is to provide an implant which overcomes difficulties outlined above. This object is solved by the implant according to claim 1.

The invention relates to the prosthesis which replaces the TMJ and makes to keep movement of the joint which cannot fulfill its task due to disorders / uncorrectable defects.

The prosthesis has got a structure that can provide surgeons to adaptation of the prosthesis to the jaw as soon as possible and in a comfortable way during surgery. We aimed to put forward a top level with regard to patient comfort and long-lasting prosthesis by minimizing friction due to pressures and stresses on moving parts.

### MEANING OF THE FIGURES

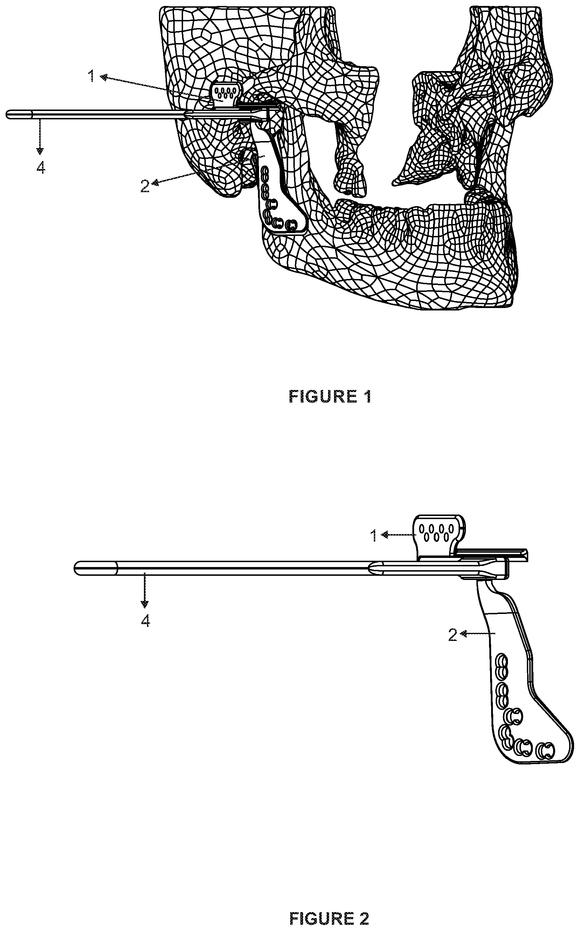

Figure 1. Perspective View on mounted skull
Figure 2. Assembled Perspective View

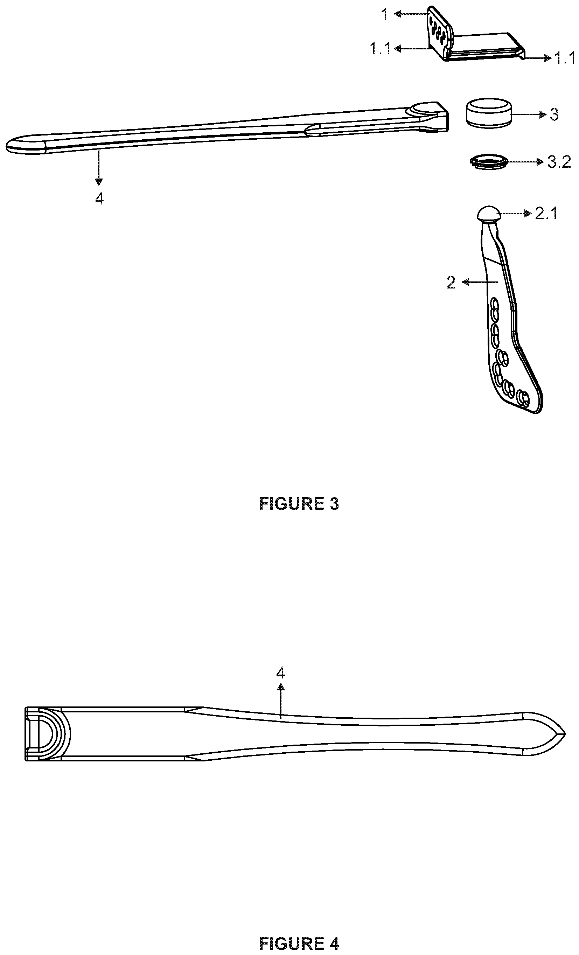

Figure 3. Exploded View
Figure 4. Bottom view of Measuring Instruments

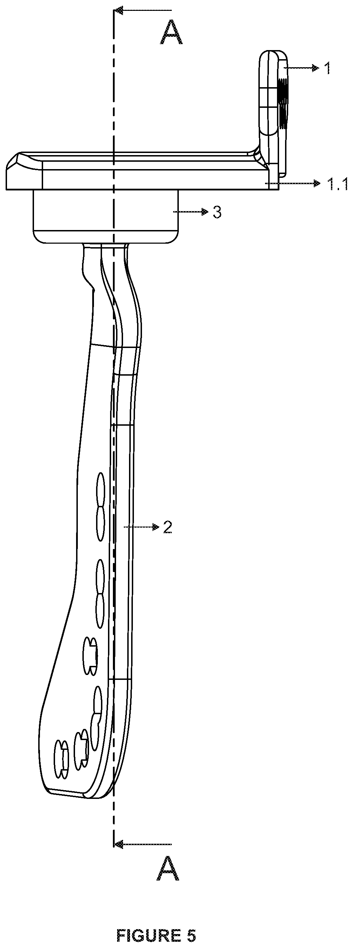

Figure 5. Assembled Front View

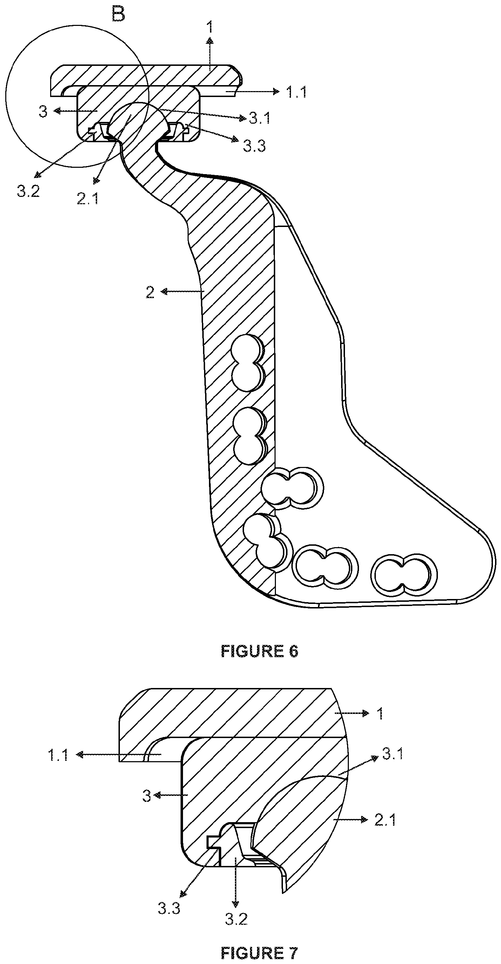

Figure 6. A-A Section View
Figure 7. B Detail View

Part number equivalents indicated in the figures are given below:
1. Fossa implant
   1.1. Stopper set
2. Apparatus condyle
   2.1. Condylar Head
3. Insert
   3.1. Condylar Head Slot
   3.2. Piston Ring
   3.3. Ring Groove
4. Gauger

### DETAILED DESCRIPTION OF THE INVENTION

Temporomandibular joint (TMJ) is one of the most complex joints of human body.

This joint is located between mandible and maxilla and responsible for digestion, and speech functions. This joint exists on both left and right side of body, has 4 joint surfaces and surrounded digestion muscles, cartilagenous tissues. There is some liquid between this joint surfaces in order to prevent friction of cartilages and to support movement. TMJ has a reel shape and complex structure, it moves both back-forward, and left-right. It has some differences than vertebra and knee joints. In normal physiological situation at 1 cm of the opening of TMJ, condyle rotates, and then moves to forward. TMJ joint is moving when chewing, swallowing, and respiration. Normally this joint is used totally 1500-2000 times per day. This movement is forward, backward, left side, and right side.

TMJ joint disorders is very common and it is one of the main reason of head and neck pains. It is more common in adults and women population and risk increases in elderly period.

Invention; includes fossa implant (1), condyle apparatus and insert placed between the fossa implant and condyle apparatus. Fossa implant (1) includes, stopper sets of maximal movements of inserts (1.1). Condyle apparatus (2) includes condyler head (2.1). Insert (3); characterized condylar head seating (3.1), snap ring (3.2), and snap ring seating (3.3).

In the application of the present invention for a patient suffering from unrecoverable defects at temporomandibular joint, the deformed parts are first removed by a surgical operation. The fossa implant (1) that can be applicable to every patient is fixed to the arc bone. Then, a patient-specific temporary condyle apparatus (2) is fixed to the mandibular. A suitable insert (3) for the patient is selected by measuring the distance between the fossa implant (1) and condylar head (2.1) with a measuring device (4). After completing the measurement, the temporary condyle apparatus (2) is removed from the mandibular. The permanent condyle apparatus (2) is inserted in to the mandibular bone. A ring (3.2) is inserted on the condylar head (2.1). Then, the condylar housing (3.1) of the patient-specific insert (3) is fitted to the condylar head (2.1) on the condylar apparatus (2). The ring (3.2) is mounted into the ring seating (3.3) with the help of a bracket. After this operation, the condylar apparatus (2) is fixed to the mandibular using screws, which completes the operation.

Functioning of the implant when the patient returned his/her daily life: the surface of the fossa implant (1) facing to the joint and the surface of the insert (3) facing to the fossa implant (1) have a contact, which enables a degree of freedom for forward and backwards movement of the mandibular, to the extent permitted by the muscles in the sagittal plane. Moreover, this feature also enables a degree of freedom in the transverse plane. The contact at which the load is transferred is a surface contact, rather than a point contact. Although the forces exerted to the mandibular are the same, the friction value reduces since the pressure between the insert (3) and the fossa implant (1) becomes small. In addition, the condylar head (2.1) on the condylar apparatus (2) is housed into the condylar head (3.1) seating of the insert (3). The structure of the condylar head seating (3.1) permits rotational movement of the condylar head (2.1). Our invention, which has three degrees of freedom due to the structure of the muscles, is capable of producing a movement as close as possible to the normal physiological movement of the mandibular. The decrease in the friction value enables the patients to be able use the prosthesis much longer in full functionality. Our invention will give comfort to the patient's daily life, by providing movement as close as possible to the normal physiological movement of the mandibular.

## Claims

1. A temporomandibular implant comprising:
a fossa implant (1),
a condylar apparatus (2) with a condylar head (2.1), and
an insert (3) having
a condylar head seating (3.1) for housing the condylar head (2.1), and having three degrees of freedom between the condylar apparatus (2) and the fossa implant (1),
**characterized in that** the temporomandibular implant further comprises a ring seating (3.3) in the insert (3) and a snap ring (3.2) adapted to be mounted into the ring seating (3.3), the condylar head seating (3.1) being adapted to fit to the snap ring (3.2) and to the condylar head (2.1), wherein the insert (3) is adapted to have a contact with the fossa implant (1) that enables a degree of freedom for forward and backwards movement of the insert (3), wherein the insert (3) is patient-specific and is selected by measuring the distance between the fossa implant (1) and the condylar head (2.1) with a measurement device (4), wherein stopper sets (1.1) are provided to control the forward and backwards movement of the insert (3).

2. The temporomandibular implant according to claim 1, wherein the condylar head (2.1) is adapted to exactly fit to the condylar head seating of the insert (3).

## Patentansprüche

1. Kiefergelenkimplantat umfassend:
ein Fossaimplantat (1),
einen Gelenkapparat (2) mit einem Gelenkkopf (2.1) und
einen Einsatz (3) mit
einer Gelenkkopfaufnahme (3.1) zum Aufnehmen des Gelenkkopfs (2.1) mit drei Freiheitsgraden zwischen dem Gelenkapparat (2) und dem Fossaimplantat (1),
**dadurch gekennzeichnet, dass** das Kiefergelenkimplantat weiterhin eine in dem Einsatz (3) ausgebildete Ringaufnahme (3.3) und einen Sicherungsring (3.2) umfasst, der dafür angepasst ist, in der Ringaufnahme (3.3) angebracht zu werden, wobei die Gelenkkopfaufnahme (3.1) dafür angepasst ist, mit dem Sicherungsring (3.2) und dem Gelenkkopf (2.1) zusammenzupassen, wobei der Einsatz (3) dafür angepasst ist, einen Kontakt mit dem Fossaimplantat (1) aufzuweisen, der einen Freiheitsgrad für eine Vorwärts- und Rückwärtsbewegung des Einsatzes (3) ermöglicht, wobei der Einsatz (3) patientenspezifisch ist und durch Abmessen der Distanz zwischen dem Fossaimplantat (1) und dem Gelenkkopf (2.1) mittels einer Messvorrichtung (4) ausgewählt wird, wobei Anschlagsätze (1.1) vorgesehen sind, um eine kontrollierte Vorwärts- und Rückwärtsbewegung des Einsatzes (3) zu gewährleisten.

2. Kiefergelenkimplantat nach Anspruch 1, wobei der Gelenkkopf (2,1) dafür angepasst ist, präzise mit der Gelenkkopfaufnahme des Einsatzes (3) zusammenzupassen.

## Revendications

1. Implant temporo-mandibulaire, comprenant :
un implant pour fosse mandibulaire (1),
un appareil articulaire (2) avec une tête d'articulation (2.1) et
un insert (3) avec
un logement de tête d'articulation (3.1) destiné à recevoir la tête d'articulation (2.1) avec trois degrés de liberté entre l'appareil articulaire (2) et l'implant pour fosse mandibulaire (1),
**caractérisé en ce que** l'implant temporo-mandibulaire comprend en outre un logement de bague (3.3) formé dans l'insert (3) et une bague de sûreté (3.2) conçue pour être placée dans le logement de bague (3.3), le logement de tête d'articulation (3.1) étant conçu pour être adapté à la bague de sûreté (3.2) et à la tête d'articulation (2.1), l'insert (3) étant conçu pour présenter un contact avec l'implant pour fosse mandibulaire (1) lequel offre un degré de liberté permettant à l'insert (3) de décrire un mouvement avant-arrière, ledit insert (3) étant adapté au patient et étant sélectionné en mesurant la distance entre l'implant pour fosse mandibulaire (1) et la tête d'articulation (2.1) au moyen d'un dispositif de mesure (4), des sets de butée (1.1) étant prévus afin de garantir un mouvement avant-arrière contrôlé de l'insert (3).

2. Implant temporo-mandibulaire selon la revendication 1, dans lequel la tête d'articulation (2.1) est conçue pour être adaptée de manière précise au logement de tête d'articulation de l'insert (3).
